# EUROPEAN PATENT SPECIFICATION

(11) **EP 2 033 528 B1**
(45) Date of publication and mention of the grant of the patent: **13.04.2016**
(21) Application number: 07020945.7
(22) Date of filing: 25.10.2007
(51) Int. Cl.: A23L 33/105, A61K 8/97, B01D 11/00, A61K 36/45

(54) **LINGONBERRY EXTRACT, THE PREPARING METHOD AND USE THEREOF**
PREISELBEERENEXTRAKT SOWIE HERSTELLUNGSVERFAHREN DAFÜR UND VERWENDUNG DAVON
EXTRAIT D'AIRELLE, PROCÉDÉ DE PRÉPARATION ET UTILISATION CORRESPONDANTE

(30) Priority: 04.07.2007 CN 200710122746
(43) Date of publication of application: 11.03.2009
(73) Proprietor: Beijing Gingko Group Biological Technology Co., Ltd., Haidian District Beijing 100081 (CN)
(72) Inventor: Li, Yanmei, Haidian District Beijing 100081 (CN); Dou, Xiarui, Haidian District Beijing 100081 (CN); Cheng, Qiaojie, Haidian District Beijing 100081 (CN)
(74) Representative: Manitz, Finsterwald & Partner GbR

(56) References cited:
- WO-A1-2006/111163
- WO-A1-2006/134583
- FR-A1- 2 887 148
- ANDERSEN: "cHROMATOGRAPHIC SEPARATION OF ANTHOCYANININ COWBERRY (LINGONBERRY) VACCINIUM VITES-IDAEA l.", JOURNAL OF FOOD SCIENCE, vol. 50, 1985, pages 1230-1232, XP002687084, ISSN: 0022-1147
- BOMSER J ET AL: "IN VITRO ANTICANCER ACTIVITY OF FRUIT EXTRACTS FROM VACCINIUM SPECIES", PLANTA MEDICA, THIEME VERLAG, DE, vol. 62, no. 3, 1 January 1996 (1996-01-01), pages 212-216, XP008021544, ISSN: 0032-0943
- DATABASE CAPLUS [Online] CHEMICAL ABSTRACTS SERVICE, COLUMBUS, OHIO, US; 5 August 2004 (2004-08-05), XP002687085, retrieved from STN Database accession no. 2004:625599 & CZ 292 834 B6 (IVAX PHARMACEUTICALS SRO [CZ]) 17 December 2003 (2003-12-17)
- DATABASE CAPLUS [Online] CHEMICAL ABSTRACTS SERVICE, COLUMBUS, OHIO, US; 2 April 2002 (2002-04-02), XP002687086, retrieved from STN Database accession no. 2002:245024 & JP 2002 097147 A (NOEVIR KK) 2 April 2002 (2002-04-02)
- DATABASE CAPLUS [Online] CHEMICAL ABSTRACTS SERVICE, COLUMBUS, OHIO, US; 20 September 2000 (2000-09-20), XP002687087, retrieved from STN Database accession no. 2000:658029 & JP 2000 256176 A (SHALOM KK) 19 September 2000 (2000-09-19)
- HEINONEN: "Antioxidant activity and antimicrobial effect of berry phenolics - a Finnish perspective", MOL. NUTR. FOOD RESEARCH, vol. 51, June 2007 (2007-06), pages 684-691, XP002687114, DOI: 10.1002/mnfr.200700006

## Description

### Field

This invention relates to lingonberry extracts.

### Background of Technology

Anthocyanin, also known as anthocyan, which belongs to flavonoid compounds, has the following physiological activities: i) increasing flexility of capillary, thus improving the swellability and retractility of blood vessels and preventing angiorrhexis; ii) eliminating free radicals which may result in blood vessels sclerosis, thus preventing angiosclerosis; and iii) strengthening capillary in eyes and other tissue organ of body, and preventing pathological changes of these organs. Anthocyanins have been used in foods and medicines. Up till now, those used in foods are anthocyanidins like pelargonidin, cyanidin, delphinidin, peonidin, petunidin, malvidin, or anthocyanins formed by these anthocyanidins binding with glucose, galactose or arabinose respectively. Anthocyanins are mostly monosaccharide and diglycoside.

A proanthocyanidin is a condensed tannin from plant sourse, which produces anthocyanins when heated in an acidic medium. It can reduce the levels of cholesterol and low-density lipoprotein, thus prevent thrombosis and cardio-cerebro-vascular disease, and protect DNA from damage of free radicals, preventing gene mutation which may cause cancer.

Resveratrol and its glucoside are a category of active polyphenol substances, and abundantly contained in a Chinese medicine named *Polygonum cuspidatum.* Apart from its strong antioxidant function, resveratrol can inhibit three major stages (induction, initiation and development) of cytometaplasia and tissue mutation in the process of canceration, and becomes one of the most prospective substances used for inhibiting and treating tissue canceration and tumorigenesis. Recently, it has been reported that resveratrol can delay ageing process by protecting DNA from chemical damage. In addition, recent experiments about organic life-span were made to the common life-increasing mechanism of resveratrol and restricting calorie ingest. It has been observed that resveratrol can increase life span by about 70% through activating Sir2 enzymic expression. Hence, resveratrol influences organism health by activating Sir2 enzyme in different levels and paths.

Therefore, using extract anthocyanins, proanthocyanidin and resveratrol from plants as drugs and health foods is consistent with market demand.

Andersen discloses in Journal of Food Science, volume 50, 1985, pages 1230 to 1232 a process for preparing an extract of lingonberry comprising the step of extracting lingonberry with acidified ethanol as a first solvent and then re-extracting the residue with acidified ethanol as second solvent.

Bomser et al. discloses in Planta Medica, volume 62, number 3, 1996, pages 212 to 216 a process for preparing an extract of lingonberry comprising the steps of extracting lingonberry with acidified methanol and then with ethyl acetetate.

WO 2006/111163 A1 describes a process for preparing an extract of lingonberry comprising the step of incubating a plant material with an enzyme composition comprising a lipolytic enzyme.

From Chemical abstracts service, Columbus, Ohio, US, accession number 2004:625599 a process for preparing an extract of lingonberry comprising the step of extracting lingonberry with a mineral acid is known.

Heinonen discloses in Mol. Nutr. Food Res., Volume 51, 2007, pages 684-691, the phenolic profiles of someberries.

### Summary

It is one of an object to provide an extract riched with anthocyanin, proanthocyanidin and resveratrol from lingonberry fruit.

Lingonberry *(Vaccinium vitis-idaea* L.) also named as red bean, an evergreen dwarf shrub with a height of 15-30cm, belongs to *Vaccinium* of Ericaceae, and is a perennial tree species of deciduous leaf or evergreen shrub or dwarf shrub. It origins in alpine areas of northeast of China, Korea, the Former Soviet Union, North America and North Europe, often living together with bog bilberry (*vaccinium uliginosum.L*). Its leaves are evergreen and herbaceous. Lingonberry has excellent cold hardiness and resistance to drought. The fruit of lingonberry is brilliant red, tastes acerbic and has high value in use.

Upon studing on lingonberry, the inventor found that the fruit of lingonberry riches in anthocyanin, proanthocyanidin and resveratrol.

Accordingly, the present invention provides:
an extract of lingonberry, which is comprised of 5-35wt% of anthocyanin, 20-95 wt% of proanthocyanidin, less than 5 wt% of resveratrol and the balance of other components.

Preferably, the extract of lingonberry is comprised of 5-20 wt% of anthocyanin, 50-95 wt% of proanthocyanidin, less than 2 wt% of resveratrol and the balance of other components.

According to one embodiment of the present invention, the anthocyanin substantively comprises cyanidin-3-galactose, cyanidin-3-glucose and cyanidin-3-arabinose. Moreover, the present invention is related to an extract composition of lingonberry, which is comprised of:
- a first extract of lingonberry comprised of 5-20 wt% of anthocyanin, 50-95 wt% of proanthocyanidin, less than 2 wt% of resveratrol and the balance of other components, and
- a second extract of lingonberry containing 60-98 wt% of resveratrol.

Preferably, the extract of lingonberry is an extract product obtainable by a process comprising the steps of :
(a) extracting the fruit of lingonberry with a first solvent selected from alcohol, aqueous alcohol solution, water, aqueous acid solution, aqueous enzyme-containing solution, or aqueous acid-alcohol solution, and then isolating to obtain first extract solution;
(b) evaporating the first extract solution to obtain a crude first extract ;
(c) purifying the crude first extract to obtain a first extract product.

According to another embodiment of the present invention, in step (a) the first solvent is an aqueous acid solution or an aqueous acid-alcohol solution containing no more than 10 wt% of hydrochloric acid, sulphuric acid, acetic acid or citric acid, or a aqueous solution containing 0.05-0.2 wt% of cellulase or pectinase.

Preferably, in step (a),
- the first solvent is an aqueous acid solution or an aqueous acid-alcohol solution, and the extraction is performed under a temperature of 20-50 °C; or
- the first solvent is an aqueous alcohol solution, and the extraction is performed under a temperature of 40-90 °C; or
- the first solvent is an aqueous enzyme-containing solution, and the extraction is performed under a temperature of 30-60 °C.

Moreover, it is preferred that in step (c),
- the crude first extract is purified with an absorptive resin or an ion exchange resin, including transferring the crude first extract into an absorptive resin column or an ion exchange resin column, eluting with 30-95% ethanol, concentrating the obtained ethanol solution and drying to obtain a first extract of lingonberry; or
- the crude first extract is purified by an ultra-filtration membrane, including passing the crude first extract through an ultrafiltration membrane, concentrating the obtained retentate or passing it by a reverse osmosis membrane, and drying the obtained concentrated solution to obtain the first extract of lingonberry; or
- the purification is performed combining the above two methods, including passing the crude first extract through an ultrafiltration membrane, purifying the obtained retentate with a resin column, and drying to obtain the first extract of lingonberry; or purifying the crude first extract by a resin column, then treating with an ultrafiltration membrane, and drying to obtain the first extract of lingonberry.

According to another embodiment of the present invention, the first extract of lingonberry comprised of 5-20 wt% of anthocyanin, 50-95 wt% of proanthocyanidin, less than 2 wt% of resveratrol and the balance of other components is obtainable by a process comprising the steps of:
(a) extracting the fruit of lingonberry with a first solvent selected from alcohol, aqueous alcohol solution, water, aqueous acid solution, aqueous enzyme-containing solution, or aqueous acid-alcohol solution, and then isolating to obtain a first extract solution;
(b) concentrating the first extract solution to obtain a crude first extract ;
(c) purifying the crude first extract to obtain a first extract; and
wherein the second extract of lingonberry containing 60-98 wt% of resveratrol is obtainable by a process comprising the steps of:
(d) re-extracting the fruit of lingonberry, which has extracted with the first solvent, with a second solvent to obtain a second extract solution, wherein the second extract solution is selected from ethyl acetate, benzene, toluene, chloroform, dichloromethane, n-hexane, acetone, methanol, ethanol, and the mixture thereof, or 60-95wt% methanol or ethanol aqueous solution, or aqueous enzyme-containing solution containing 0.01-0.3 wt% of cellulase or pectase;
(e) concentrating the second extract solution to obtain a crude second extract;
(f) purifying the crude second extract to obtain a second extract of lingonberry in which the main component is resveratrol.

In the aforementioned embodiment, it is preferred that
- in step (a) the first solvent is an aqueous acid solution or an aqueous acid-alcohol solution, and the extraction is performed under 20-50 °C; or the first solvent is aqueous alcohol solution, and the extraction is performed under 40-90 °C; or the first solvent is the enzyme aqueous solution, and the enzymolysis is performed under the temperature of 30-60 °C;
- in step (b), the first extract solution is concentrated to obtain the crude first extract;
- in step (c), the crude first extract is transferred into an absorptive resin column or an ion exchange resin column, eluted with 30-95% ethanol, wherein the obtained ethanol solution is concentrated and dried to obtain a first extract of lingonberry; or
   the crude first extract is purified by an ultra-filtration membrane, including passing the crude first extract through an ultrafiltration membrane, concentrating the obtained retentate or passing it by a reverse osmosis membrane, and drying the obtained concentrated solution to obtain the first extract of lingonberry; or the purification is performed combining the above two methods, including passing the crude first extract through an ultrafiltration membrane, purifying the obtained retentate with a resin column, and drying to obtain the first extract of lingonberry; or purifying the crude first extract by a resin column, then treating with an ultrafiltration membrane, and drying to obtain the first extract of lingonberry;
- in step (d), the re-extracting is performed under light-proof and inert atmosphere to obtain the second extract solution;
- in step (e), the second extract solution is concentrated to obtain the crude second extract;
- in step (f), the crude second extract is purified by silica gel column chromatography with n-hexane, ethyl acetate, acetone, chloroform, dichlormethane, methanol, ethanol or a mixture solvent thereof to obtain the second extract of lingonberry.

The products according to the present invention can be used as functional foods for anti-aging, weight loss, eliminating oxygen free redicals, preventing cardiovascular and cerebrovascular diseases, inhibiting thrombosis, and beauty treatment.

### Brief Description of the Drawings

Fig.1 is a HPLC spectrum of the first extract obtained in one embodiment of the invention;
Fig.2 is a HPLC spectrum of the second extract obtained in one embodiment of the invention;
Fig.3 is a HPLC spectrum of the third extract obtained in one embodiment of the invention.

### DETAILED DESCRIPTION OF THE INVENTION

### Definitions

The term "anthocyanin" refers to a chromene derivate making the flower, fruit, leaf, stem etc. of plants to present notable color such as blue, purple, red etc., most of which can condensate with glucose, galactose or rhamnose and some pentose to form anthocyanin. Typical anthocyanin includes pelargonidin, cyanidin, delphinidin, peonidin, petunnidin, malvidin, hireutidin and gesnerin.

The term "proanthocyanidin" refers to a category of polyphenol compounds which exsit in plants and result in anthocyanin when heated in acidic mediums.

In the first aspect of the invention, it provides a first extract of lingonberry which is substantially comprised of anthocyanin, proanthocyanidin and resveratrol. More particularly, the first extract according to the first aspect of the invention is comprised of 5-35 wt% of anthocyanin, 20-95 wt% of proanthocyanidin: less than 5 wt% of resveratrol and the balance of other components. Said other components refer to eatable substances that are extracted out together with the expected components under the extraction condition described herein. The first extract of lingonberry obtained according to a particular method of the present invention contains 5-20 wt% of anthocyanin, 50-95 wt% of proanthocyanidin, less than 2 wt% of resveratrol and the balance of other components, in which Anthocyanin mainly includes cyaniding-3-galactose, cyaniding-3-glucose and cyaniding-3-arabinose, and proanthocyanidin mainly includes proanthocyanidin B1, proanthocyanidin B2, and proanthocyanidin C1.

According to the second aspect of the invention, it provides a composition of lingonberry extracts, comprised of, at any weight ratio, the first extract described above and a second extract, wherein the second extract of lingonberry contains 60-98 wt% of resveratrol. In one embodiment of the invention, the second extract of lingonberry contains 75-85 wt% of resveratrol. In one example of the invention, the second extract of lingonberry contains 80 wt% of resveratrol.

Anthocyanin and proanthocyanidin belong to polyhydroxy flavone. Therefore, all extraction methods in the prior art to extract polyhydroxy flavone can be used in the present invention in principle. The methods for preparing the first extract of lingonberry usually include extraction and purification steps. Herein, "extraction" refers to a process for abstracting objective components from raw material of lingonberry by means of physical and/or chemical methods; and "isolation/isolating" or "purifica tion/purifying" refers to isolating objective components from a crude extract through physical methods basically. The extraction method can be solvent extraction, ultrasonic extraction, enzymic extraction and supercritical fluid extraction. The preferred methods in this invention are solvent extraction method and enzymic extraction method.

There are various kinds of methods for isolating anthocyanin and proanthocyanidin from the crude first extract. These methods include the ones frequently used in the prior art for isolating the flavone compounds, such as solvent extraction method, alkali-extraction and acid-precipitation method, polyamide column chromatography, silica gel column chromatography, boracic acid complexation method, pH gradient extraction method and gel column chromatography. These methods were described in 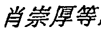 , 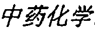 . 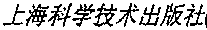 *(*Xiao Chonghou et al., Traditional Chinese Medicinal Chemistry, Shanghai Science and Technology Press), 1994, 265*,* and 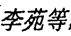 , 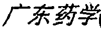 *(*Li Yuan et al., Guangdong Pharmacy), 1999, 9(2), P4*.* A skilled in the art can easily find suitable method for isolating anthocyanin and proanthocyanidin from above crude extract according to the teachings of above methods as well as their own experiences. The preferred methods of this invention are membrane filtration method and adsorption resin method.

In one embodiment of the invention, extracting the first extract of lingonberry is performed by using a first solvent, which is an alcohol, an aqueous alcohol solution, water, an aqueous acid solution, or an aqueous acid-alcohol solution, and the extraction method may be any of the followings:

### 1) Water extraction

Hot water extraction is usually used to extract various flavonoid glycosides, during which a raw material is placed into hot water or even boiling water to damage enzymatic activity. Usually, one should take into account the water amount, soaking time, extraction time and extraction times. Usually, it is feasible to use 1-5 folds of water, preferably 1-3 folds of water, which may be heated to 30-100 °C, preferably 30-80 °C, more preferably 30-60 °C and the most preferably 40-50 °C. The extraction time can be varied according to the temperature. That is to say, extraction time can be reduced under a higher temperature and longer accordingly under a lower temperature. But in view of producting efficiency, the extraction time is usually controlled within 24 hours, preferably within 16 hours, more preferably within 8 hours, and the most preferably within 4 hours, e.g. within 1 or 2 hours. Additionally, the above extraction process may be repeated several times. Generally, extraction time, temperature and times should be considered interactively, for example, extraction may be done fewer times or even one time under a higher temperature or for a longer extraction time. In one embodiment of the invention, the extraction is repeated twice with 3-5 fold water, under 60-80 °C for 16 hours. Preferably, a small amount of inorganic acid or organic acid may be added into the water, and "small amount" refers to the amount of the acid is no more than 10% by weight, preferably no more than 5% by weight and more preferably no more than 3 % by weight herein. But the amount should be enough to adjust pH of aqueous solution to no more than 3, e.g.1. There is no strict limit about the acid used herein, and hydrochloric acid and sulfuric acid which have lower cost are preferred. Other preferred acids include acetic acid, oxalic acid , malonic acid, butanedioic acid and citric acid. In the case of using acid, extraction may be performed under ambient temperature such as 20 °C. In one embodiment of the invention, extraction is performed 1-2 times in 3-5 fold water, at pH in the range of from 1 to 2, under 40-50 °C for 60-90 minutes.

### 2) Ethanol or methanol extraction

Ethanol or methanol is the most common extractant for flavone compounds. High concentration of aqueous alcohol solution (e.g. of 90-95wt%) is suitable for extracting aglycones; and alcohol of about 60wt% is suitable for extracting glycosides. The extracting time can be varied according to the temperature. That is to say, extraction time can be reduced under a higher temperature and longer accordingly under a lower temperature. But in view of product efficiency, the extraction time is usually controlled within 16 hours, preferably within 10 hours, more preferably within 6 hours, e.g. 1 or 2 hours. Additionally, the above extraction process may be repeated several times, e.g. repeating 2-4 times. Generally, extraction time, temperature and times should be considered interactively, for example, extraction may be done fewer times or even one time under a higher temperature or for a longer extraction time. Usually, aqueous alcohol solution with alcohol content in the range of 30-70 wt% is suitable. In one embodiment of the invention, the extraction is performed twice in 3-5 fold methanol aqueous solution, under 60-70 °C for 90 minutes for each time.

In the present invention, enzymic method is also used for extracting anthocyanin and proanthocyanidin components, the examples of enzyme which can be used include glucose oxidase and cellulase. The examples of extracting flavone compounds by enzymic methods were disclosed in 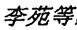 , *«* 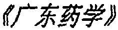 *» (*Li Yuan etc., Guangdong Pharmacy), 1999, 9 (2): 4*;* 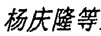 *, «* 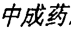 *» (*Yang Qinglong et al., Chinese Traditional Patent Medicine), 1995, 17 (6): 4*;* and 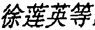 *, «* 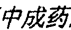 *» (*Xu Lianying et al., Chinese Traditional Patent Medicine), 2001,32(1):37*,* which are introduced into the present invention by reference. In the examples of the invention, the enzymolysis condition were chosen as aqueous solvent containing 0.05-0.2 wt% of cellulase or pectinase, enzymolysis temperature of 30-60 °C and the ethanol concentration of 60%.

In addition, the extraction of anthocyanin and proanthocyanidin is also performed by membrane filter method including: isolating the fruit extract of lingonberry by ceramic membrane separation, passing the permeate through an ultrafiltration membrane filter and then isolating the dialysate by a reverse osmosis membrane(or nanofiltration membrane). Examples for isolating flavone and polyphenol compounds by membrane filtration method were disclosed in 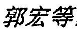 , 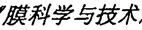 *» (*Guo Hong etc., Membrane Science and Technology), 2003, 23 (4): 197; Japanese patent WO 01/001798 disclosed a method for extracting anthocyanin by membrane-filtering from polyphenol-rich substances, which is incorporated into the present invention by reference.

Subsequently, the second extract of lingonberry is extracted with a second solvent. Generally, the polarity of the second solvent is lower than that of the first solvent. The suitable second solvent can be selected from ethyl acetate, benzene, toluene, chloroform, dichloromethane, n-hexane, acetone, methanol, ethanol or mixture thereof, or 70-95wt% aqueous methanol or ethanol solution.

In an embodiment of the invention, ethyl acetate is used. In another embodiment, 70-95wt% ethanol aqueous solution is used. In another embodiment, n-hexane is used. Under the later condition, the yield of the products may be reduced, but the content of resveratrol in the extract is relatively higher.

In practice, one may extract lingonberry fruit firstly using the first solvent to separate out the first extract of lingonberry, and then use the second solvent to re-extract the raw material which has been extracted by first solvent, to obtain the second extract solution of lingonberry The extraction order may also be reversed. But the above first order is preferred.

In the invention, the purification step may be performed by using a macroporous adsorption resin or ion exchange resin column chromatography, silica gel column chromatography, ultrafiltration or combination of the above methods. Macroporous resins that may be used include, without limitation, HP-20, ADS-F8, HPD-500, YWD-06, SP850, HP-30, S-861, D-101, DA-201, GDX-105, MD05271, CAD-40, XAD-4, XAD-6, and so on. The ion exchange resin could be used including, but not limiting to, CHPA-20, WK1, CK08S, SP-550C, DEAE-650S and SP-5PW.

Silica gel column chromatography is well known in the art. In one embodiment of the invention, this method is used for purifying the second extract of lingonberry. The elution solvent is selected according to the properties of resveratrol. The solution used may be n-hexane, ethyl acetate, acetone, chloroform, dichloromethane, methanol, ethanol or a mixture thereof. Isolating resveratrol by silica gel column chromatography is also well known in the art, so the details of the same are not given herein.

A drying step, preferably a vacuum drying is necessary after the purification step.

In the case of the solvent extraction, preferably, lingonberry raw material is exacted firstly with the first solvent and the first extract solution is filtered out, then the solid residue is extracted with the second solvent and the second extract solution is filtered, and then the first extract solution and the second extract solution are treated to obtain the first extract and the second extract of lingonberry respectively.

Therefore, are prepared by the above extracts of lingonberry following steps:
(a) extraction: extracting new lingonberry raw material with the first solvent to obtain the first extract solution of lingonberry;
(b) purification: purifying the first extract solution obtained in step (a);
(c) drying: drying the purified substance obtained in step (b) to obtain the first extract of lingonberry;
(d) extraction: re-extracting the raw material, which has been extracted with the first solvent, with the second solvent to obtain the second extract solution of lingonberry;
(e) purification: purifying the second extract solution to obtain the second extract of lingonberry (high content of resveratrol).
(f) mixing the first extract and the second extract at any ratio to obtain an extract composition of lingonberry.

In step (f), the ratio of first extract and second extract is designed according to the application of an objective product. For example, it may contain more of the first extract or even contain no second extract for the purpose of antioxidant. It may contain the first extract dominantly and a proper proportion of the second extract if used for anti-aging, e.g. the first extract/the second extract may be 10 : 1. The composition may contain the first extract and the second extract at substantially the same quantity for the purpose of weight loss.

In an embodiment, the above method contains the following steps:
(a) extraction: extracting lingonberry fruit with a hot aqueous acid solution, or an aqueous alcohol solution or an aqueous enzyme-containing solution, and then filtering to obtain the first extract solution;
(b) purification: purifying the first extract solution with a resin or an ultrafiltration membrane;
(c) drying: drying the purified substance obtained in step (b) to obtain the first extract of lingonberry;
(d) extraction: extracting the filtered residue, which has been extracted with acid aqueous solvent (or aqueous alcohol solution or enzyme aqueous solution), with the second solvent to obtain the second extract solution;
(e) purification: purifying the second extract solution to obtain the second extract of lingonberry (high content of resveratrol);
(f) mixing the first extract and the second extract at the ratio of 0.001:100-100:0.001 to obtain the third extract of lingonberry.

In a more particular embodiment of the method, the method for preparing the above extracts of lingonberry contains following steps:
(a) extraction: extracting lingonberry fruit with hydrochloric acid, sulphuric acid or aqueous acetic acid solution of 1-5 wt%; or methanol or ethanol aqueous solution (preferred of 30-70 wt%); or cellulase or pectinase aqueous solution of 0.05-0.2 wt%, and filtering under a reduced pressure to obtain the first extract solution;
(b) purification: concentrating to substantially remove the solvent, transferring the concentrated solution onto a macroporous adsorption resin or an ion exchange resin column and, after washing it to neutrality, eluting it with 30-95 wt% ethanol; or performing a purifying treatment with a ultrafiltration membrane; or performing said purification by combining the above two methods, including filtering said crude first extract though an ultrafiltration membrane, purifying the retentate with the resin column, and then drying to obtain the first extract of lingonberry, or alternatively, purifying said crude first extract with a resin column, filtering the eluate with an ultrafiltration membrane filter and then drying to obtain the first extract of lingonberry ;
(c) drying: vacuum concentrating the ethanol solution obtained in step (b) and drying to obtain the first extract of lingonberry;
(d) extraction: extracting the residue, which is filtered in step (a), with ethyl acetate, n-hexane, acetone, methanol, ethanol, or their aqueous solution to obtain the second extract solution. This step may be performed 1-2 times under light-proof and inert atmosphere;
(e) purification: purifying the second extract solution obtained in step (d) by silica gel column chromatography with a solvent selected from n-hexane, ethyl acetate, acetone, methanol, ethanol or the mixture thereof in order to obtain the second extract of lingonberry (high content of resveratrol);
(f) mixing the first extract and the second extract at a ratio of 0.001: 100-100: 0.001 to obtain the third extract of lingonberry.

### Analysis:

In the present invention, the analysis of the first extract and the second extract of lingonberry and the composition thereof is performed by high performance liquid chromatography (HPLC).

For the first extract, the chromatographic condition (Condition 1) used is:
chromatographic column: ODS-C18
gradient mobile phase : phase A, water : formic acid = 90 : 10
   phase B, water:acetonitrile:methanol =45:22.5:22.5
wave length: 540nm

Or Condition 2 is used:
chromatographic column: ODS-C18
gradient mobile phase: phase A, acetonitrile
   phase B, phosphoric acid : water =67 : 31
wave length: 280nm

For the second extract, the chromatographic condition (Condition 3) used is:
chromatographic column: ODS-C18
gradient mobile phase : phase A, acetonitrile
   phase B, water: formic acid = 270 : 1
wave length: 303nm

Additionally, in the present invention, quantitative analysis of anthocyanin and resveratrol is performed by HPLC, and for analysis of proanthocyanidin, we employed a testing method in the art suitable for polyphenol of grapeseed-the method of potassium sodium tartrate, i.e., using the specific reaction of polyphenol with tartaric acid iron in a phosphate buffer solution at pH=7.5, and then quantitating the reaction product at a maximum absorption wavelength 540nm to obtain the sum of the polyphenols, and subtracting the amount of anthocyanin determined by HPLC from the sum to obtain the amount of proanthocyanidin.

### Applications:

The main pigment components in the extract of lingonberry are anthocyanin and proanthocyanidin, wherein anthocyanin is essentially cyanidin-3-galactoside. Anthocyanin is an antioxidant. Proanthocyanidin can be decomposed into anthocyanin in aqueous acid solution with heating, efficiently reduce the level of cholesterol and low density lipoprotein, and prevent from thrombosis and cardiovascular and cerebrovascular disease, and it also protects DNA of cell from oxidant damage of free radical and thus prevents gene mutation which may cause cancer.

The extract of lingonberry also contains resveratrol and resveratrol glucoside. Resveratrol (glucoside) is a category of active polyphenol, besides its intense antioxidant function, it has been recognized as one of the most prospective substances for inhibiting and treating tissue canceration and tumorigenesis because of their ability to inhibit three major stages (induction, initiation and development) of cytometaplasia and tissue mutation. Recently, it has been reported that resveratrol can delay ageing process by protecting DNA (deoxyribonucleic acid) in chondriosome of a cell from chemical damage. In addition, recent experiments on organic life-span were made to the common life-increasing mechanism of resveratrol and restricting calorie ingest. It has been observed that resveratrol can prolong the life span of yeast by about 70% through activating Sir2 enzymic expression. Hence, resveratrol influences organism health in multi-strata and multi-paths by activating Sir2 enzyme.

The scope of the present invention also includes the use of the extract of lingonberry in anti-aging, reducing weight, beauty treatment and treating cardiovascular and cerebrovascular disease.

Upon studying with the aid of ortho-phenanthroline Fe²⁺ oxidation method, the inventor found that the extract of lingonberry can remove hydroxy radical and its potency shows an obvious positive relationship with its concentration. Its IC₅₀ is much less than that of antiscorbic acid (Vc) under the same experiment conditions. It can be concluded that lingonberry extract has greater scavenging capability than ascorbic acid, and it has significant anti-oxidation effect *in vitro.*

Free radicals play an important role in aging and various diseases. Hence, it can be expected that the extracts of lingonberry obtained according to the method of the present invention have effects and functions in anti-aging, weight loss, beauty of whitening and anti-wrinkle and treating cardiovascular disease due to their good antioxidation ability.

Free radicals are generated continuously in the process of organism cell metabolism. Also, other external stimulating factors such as ultra-violet ray and chemical substance, etc. facilitate the generation of free radicals. The excessive accumulation of these free radicals will damage biomacromolecule substances, such as DNA and protein, thereby leading to aging of organisms. Anti-oxidation substances such as anthocyanin, proanthocyanidin and resveratrol abundantly contained in lingonberry extracts can remove efficiently free radicals and further play an important role in anti-aging. Experiments confirmed that the aged mice fed with lingonberry extract exhibit a substantially lower level of MDA, a degradation product of lipid peroxide in blood, and a higher activity of glutathione peroxidase (GSH-Px) in blood serum. It can be concluded that the lingonberry extract can act against aging through its antioxidation.

Upon treating nutritional obese rats ( resulting from high-lipid diet) with the extract of lingonberry according to the invention, the inventor found that the obese rats showed a slower increase in body weight, whereas their diet consumption were not influenced. In addition, it was found that the fat around their testes and kidneys remarkably decreased. It can be seen from the above that the extract of lingonberry can inhibit the fat accumulation in body while having no obvious appetite inhibition. Therefore, the extract can be used as a healthy diet food.

It is reported ( 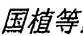 , 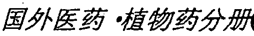 (Guo Zhi, etc.Overseas Medicine·Plant Amedica Fascicule), 1996; 11(5):196∼204) that proanthocyanidin inhibits platelet aggregation by inhibiting thromboxane TXA2. Experiments ( 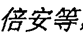 , 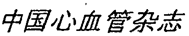 (You Beian, et al., Chinese Journal of Cardiovascular Medicine). 2003; 8(6): 383-385) demonstrate that grape seed proanthocyanidin inhibit remarkably the oxidation of low density lipoprotein in blood plasma by its anti-oxidation property and have significance in preventing and treating atherosclerosis, although they cannot influence obviously blood fat level in rabbits suffering from experimental hyperlipoidemia. Research showed that proanthocyanidin in grape seed have a relaxation effect on ex vivo aortic annulus of rabbits and the effect is relative to their facilitation to NO release of endotheliocytes *(* 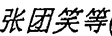 *(Zhang Tuanxiao, et al),* 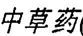 *(*Chinese Traditional and Herbal Drugs), 2006; 37 (1): 87∼89*).* The first extract of lingonberry according to the present invention riches in anthocyanin, proanthocyanidin and a small amount of resveratrol, therefore possessing good function of inhibiting thrombosis, reducing blood fat and relaxing blood vessel, and can be used as functional foods for preventing and treating high blood pressure, atherosclerosis and thrombosis disease etc.

In addition, the extracts of lingonberry can be used as beauty improvement products due to their anti-oxidation activity. Skin is a connective tissue, in which collagen protein and elastin play an important role in skin texture. Moderate cross linking of collagen protein can maintain the integrity of skin. However, the free radical oxidation in vivo may result in its over cross-linking which appears as wrinkle and vesicle on the skin. Anthocyanin and proanthocyanidin promote its moderate cross linking between collagen proteins by removing free radical efficiently, thus keeping skin's pliant, smooth and elastic. On the other hand, free radicals or elastase can degrade elastin, whilst anthocyanin and proanthocyanidin can eliminate free radicals, block generation of elastase and inhibit the activities thereof, thereby improving the health status of skin. Upon determination to the content of hydroxyproline and SOD activity in mouse skin photo-aging model constructed by exposing to ultra-violet ray, it was found that the content of hydroxyproline and SOD activity in the skin of the mice that had been fed with the extract of lingonberry are remarkably higher than that of the control animals. Consequently, the extract of lingonberry, due to its good anti-oxidation, can protect collagen protein in skin, thereby exerting its facial anti-wrinkle beauty property.

### Example 1: Preparation of the first extract of lingonberry

1000g of lingonberry fruit was extracted with hydrochloric solution of pH 1. The extract solution obtained was transferred onto a resin column (HP20 macroporous adsorption resin) and eluted with 95% aqueous ethanol. The eluant was collected , vacuum concentrated at 50°C, and then spray dried to obtain about 8g of the first extract of lingonberry.

The extract of lingonberry obtained contained 36.8% of anthocyanin, 52.7% of proanthocyanin and 3.2% of resveratrol.

### Example 2: Preparation of the first extract of lingonberry

1000g of lingonberry fruit was extracted with 80% ethanol aqueous solution. The extract solution obtained was transferred onto a resin column (CHPA20 ion exchange resin) and eluted with 60% aqueous ethanol The eluant was collected, vacuum concentrated and then spray dried to obtain about 9.5g of the first extract of lingonberry.

The first extract of lingonberry obtained contained 19.6% of anthocyanin, 72.0% of proanthocyanidin and 1.4% of resveratrol.

### Example 3: Preparation for the first extract of lingonberry

1000g of lingonberry fruit was extracted with 60% ethanol aqueous solution under 60 °C for 90 minutes. The extract solution was concentrated to remove ethanol. After diluted with water, the residue was transferred to a resin column (using HP20 macroporous adsorption resin), and eluted with 95% ethanol. The eluant was collected, vacuum concentrated and then spray dried to obtain about 8.1 g of the first extract of lingonberry.

The first extract of lingonberry obtained contained 24.5% of anthocyanin, 67.7% of proanthocyanidin and 1.5% of resveratrol.

### Example 4: Preparation of the first extract of lingonberry

1000g of lingonberry raw material was extracted using 30% ethanol aqueous solution at pH of 3. The extract solution was transferred onto a resin column (using HP20 macroporous adsorption resin) and eluted with 95% ethanol. The eluant was collected, concentrated and then spray dried to obtain about 7.6g of the first extract of lingonberry.

The extract of lingonberry obtained contained 27.8% of anthocyanin, 68.9% of proanthocyanidin and 0.7% of resveratrol.

### Example 5: Preparation of the first extract of lingonberry

1000g of lingonberry raw material was extracted by 3-fold amount of 0.08% pectinase water solution. The mixture was incubated at 40 °C for 2 hours, filtered to obtain an extract solution. The extract solution was treated with macroporous adsorption resin to obtain about 6.3g of the first extract of lingonberry.

The extract of lingonberry obtained contained 34.8% of anthocyanin, 53.9% of proanthocyanidin and 1.2% of resveratrol.

### Example 6: Preparation of the first extract of lingonberry

1000g of lingonberry fruit was extracted by 3-fold amount of 0.1% pectinase water solution. The mixture was incubated at about 40 °C for 2 hours, and then filtered to obtain an extract solution. The extract solution was filtered through an ultrafiltration membrane filter (molecular weight cut-off range of 2000). The retentate was purified by CHPA20 ion exchange resin, eluted with30% ethanol concentrated and spray dried to obtain about 8.5g of the first extract of lingonberry.

The extract of lingonberry obtained contained 5.2% of anthocyanin, 94.0% of proanthocyanidin and 0.5% of resveratrol.

### Example 7: Preparation of the first extract of lingonberry

1000g of lingonberry raw material was soaked in and extracted with 3-fold amount of pure water, and the extract solution was filtered through a ceramic membrane filter (molecular weight cut-off range of 150,000). The obtained permeate was further filtered through an ultrafiltration membrane filter (molecular weight cut-off range of 2000). And the retentate was treated by concentrated by reverse osmosis membrane filter or spray-dried directly to obtain about 15.9g of the first extract of lingonberry.

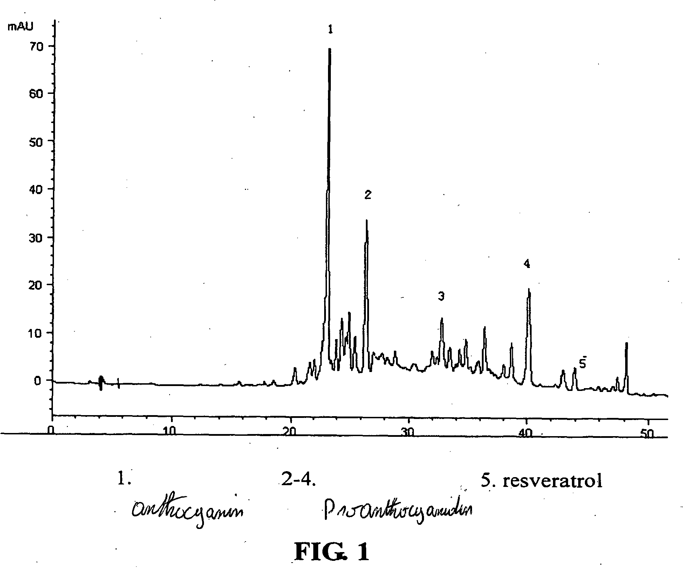

The extract of lingonberry obtained contained 5.2% of anthocyanin, 22.3% of proanthocyanidin and 0.5% of resveratrol. Fig.1 shows the HPLC spectrum of the first extract in this example using the above chromatographic Condition 1.

### Example 8: Preparation of the second extract of lingonberry

The fruit raw material (200g), which had been extracted in example 2, was further extracted with ethyl acetate under light-proof and nitrogen gas atmosphere to obtain 9.8g of yellowish paste. The paste was transferred onto silica gel column, washed with n-hexane to remove impurities, and then gradiently eluted with a mixture of n-hexane and ethyl acetate. This procedure was monitored by TLC analysis. The resveratrol-rich fraction was collected to obtain a resveratrol crude product containing 80wt% of resveratrol. The crude product was recrystallizated with ethanol to obtain 1.8g of resveratrol crystal, the second extract of lingonberry containing 98% of resveratrol.

### Example 9: Preparation of the second extract of lingonberry

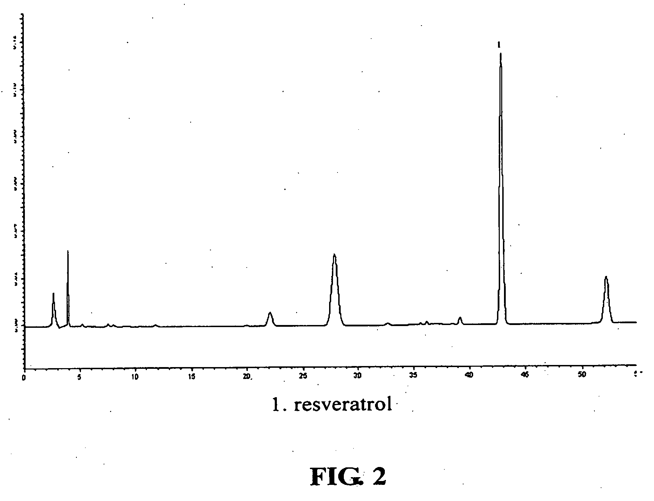

The fruit raw material (200g), which had been extracted in example 2, was further extracted with 800ml 95% ethanol under light-proof and nitrogen gas atmosphere to obtain a light yellow paste. The paste was transferred into silica gel column, washed with n-hexane to remove impurities, and then gradiently eluted with a mixture of n-hexane and ethyl acetate. This procedure was monitored by TLC analysis. The resveratrol-rich fraction was collected to obtain resveratrol crude product containing 50wt% of resveratrol. The crude product was recrystallizated with ethanol to obtain 3.1g of resveratrol crystal, the second extract of lingonberry containing 65wt% of resveratrol. Fig. 2 shows its HPLC spectrum.

### Example 10: Preparation of the second extract of lingonberry

The fruit raw material (200g), which had been extracted in example 2, was further extracted with n-hexane under light-proof and nitrogen gas atmosphere. The extract solution was transferred into a silica gel column directly, washed with n-hexane to remove impurities, and then gradiently eluted with a mixture of n-hexane and ethyl acetate. This procedure was monitored by TLC analysis. The resveratrol-rich fraction was collected to obtain resveratrol crude product containing 70wt% of resveratrol. The crude product was recrystallizated with ethanol to obtain 2.0g of resveratrol crystal, the second extract of lingonberry containing 80wt% of resveratrol.

### Example 11: Preparation of the third extract of lingonberry

0.89g of the first extract (in Example 3, containing 24.5% of anthocyanin, 67.7% of proanthocyanidin and 1.5% of resveratrol) and 0.11g of the second extract (in Example 10, containing 80% of resveratrol) were mixed, dissolved in ethanol, vacuum-concentrated and spray-dried to obtain 1.00g of the third extract (containing 21.8% of anthocyanin, 60.2% of proanthocyanidin and 10.0% of resveratrol).

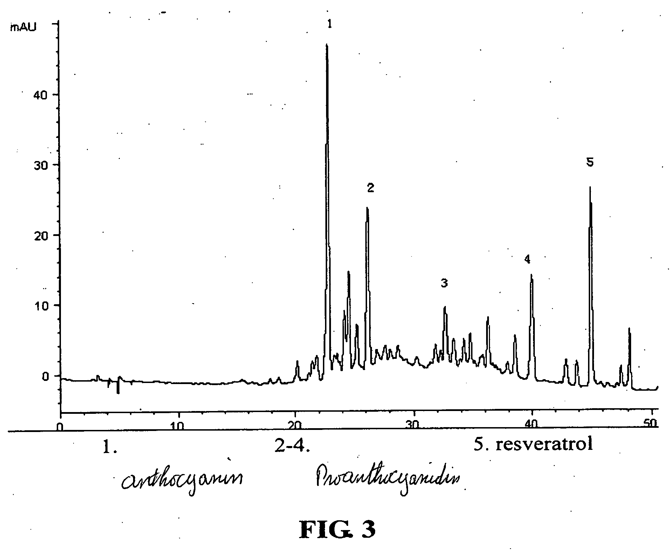

Fig.3 shows the HPLC pectrum of the third extract in this example using the above chromatographic Condition 2.

### Example 12: In vitro anti-oxidation of the first extract of lingonberry

The first extract of lingonberry (in Example 7, containing 5.2% of anthocyanin, 22.3% of proanthocyanidin and 0.5% of resveratrol) was dissolved in double-distilled water and diluted into solutions in serious concentrations of 0.5, 0.25, 0.125, 0.0625, 0.03125, 0.015625, 0.0078125mg/ml respectively. Ascorbic acid was likewise diluted to series concentrations of 2, 1, 0.5, 0.25, 0.125, 0.0625 mg/ml.

The anti-oxidation effect of lingonberry was confirmed by determining the hydroxy radical (·OH) and the scavenging effect of the test drug on the hydroxy radical using orthophenanthrolin Fe²⁺ oxidation method.

The total reaction volume is 4.7 ml, thus the final concentrations of ascorbic acid solution were 0.851, 0.426, 0.213, 0.106 mg/ml respectively and the final concentrations of the extract of lingonberry were 0.106, 0.053, 0.027, 0.013 mg/ml. Five samples in each group were determined and the scavenging rates of ·OH were calculated respectively and the average value calculated.

The results were shown in Table 1:

**Table 1: Scavenging effect of ascorbic acid on hydroxy radical (n=4)**

| final concentration (mg/ml) | ·OH scavenging rate (d) |
|---|---|
| 0.851 | 91.92±0.77 |
| 0.426 | 40.49±1.33 |
| 0.213 | 25.55±2.10 |
| 0.106 | 24.44±2.32 |

In order to verify the reliability of this method, ascorbic acid, a known ·OH scavenging agent was used as positive control when evaluating the scavenging effect of the extract of lingonberry on hydroxy radical during the experiment. The IC₅₀ was 0.3078 mg/ml.

**Table 2: Scavenging effect of the first extract of lingonberry on hydroxy radical (n=4)**

| final concentration (mg/ml) | ·OH scavenging rate (d) |
|---|---|
| 0.106 | 57.30±1.77 |
| 0.053 | 25.00±4.65 |
| 0.027 | 23.56±1.66 |
| 0.013 | 19.91±3.98 |

The IC₅₀ of the scavenging the extract of lingonberry on hydroxy radical was 0.1181mg/ml.

In the reaction system of the present experiment, the aqueous solution of the extract of lingonberry can bind with hydroxy radical generated in Fenton reaction thus reducing oxidation of Fe²⁺ by hydroxy radicals. And its effect on scavenging hydroxy radical was evaluated by determining of the absorbance value of the reaction system.

The extract of lingonberry may bind with hydroxy radical, reduce oxidation of Fe²⁺ by hydroxy radicals and increase the concentration of orthophenanthrolene-Fe²⁺ conjugates in the reaction system, thereby exerting the scavenging effect on hydroxy radicals. The scavenging capability has dose-effect relationship with the drug concentration, in that the scavenging rate has obvious positive correlation with concentration. Its IC₅₀ is 0.1181 mg/ml, whereas IC₅₀ of ascorbic acid (Vc) is 0.3078mg/ml under the same experiment conditions. Consequently its capability of scavenging hydroxy radical is stronger than ascorbic acid.

### Example 13: Influence of the extract of lingonberry on MDA content and GSH-Px activity in mouse's blood

60 ten-month-old mice (*Mus musculus* KM), after one week's adaptable breeding, were randomly divided into 4 groups : control group, test group (high, middle and low dose of lingonberry extract). The high, middle and low dose groups were administrated orally with 200, 100 and 50 mg/kg bw of the first extract of lingonberry (prepared according to example 7, wherein contains 5.2% of anthocyanin, 22.3% of proanthocyanidin and 0.5% of resveratrol) respectively, and the control group was administrated with distilled water of the same volume. The mice were killed after 8 weeks to determine the lipid peroxide degradation product MDA and glutathione peroxidase (GSH-Px) in serum. The results showed that the extract of lingonberry decreased remarkably the MDA content in old mice and improved the activity of glutathione peroxidase (GSH-Px) in serum of old mice (see Table 3).

**Table 3: The influence of the extract of lingonberry on MDA content and the activity of GSH-Px**

| | MDA (nmol/ml) | GSH-Px (U/g) |
|---|---|---|
| Control Group | 6.32±1.12 | 59.48±4.41 |
| Low Dose Group | 6.12±0.91 | 69.52±6.23 |
| Middle Dose Group | 5.42±0.89* | 75.48±6.27* |
| High Dose Group | 5.12±0.94* | 83.37±7.28* |

| | | |
|---|---|---|
| *compared with control group, P<0.05 | | |

### Example 14: Influence of the extract of lingonberry on nutritional obese rats

50 SD male rats weighing 150~200g were randomly divided into 5 groups according to body weight after adaptable breeding with normal feed for one week: normal feed group, high fat feed group, test groups, i.e. high dose group, middle dose group and low dose group, the normal feed group fed with common feed, while the other groups fed with high fat feed. The high fat feed Group was composed of 55% of common feed, 12% of lard, 5% of sucrose, 8% of milk powder, 5% of peanut, 10% of eggs, 3% of sesame oil and 2% of salt. The high, middle and low dose groups were respectively administrated with 200, 100 and 50 mg/kg bw of the first extract of lingonberry (prepared according to example 7, containing 5.2% of anthocyanin, 22.3% of proanthocyanidin and 0.5% of resveratrol), while control group was administrated with distilled water of the same volume. The body weight and food-intake of the test animals were recorded on each day. The animals were killed after 8 weeks, the weights of the adipose tissue around their kidneys and testes were recorded.

In the course of the experiment, all rats grew well, that is to say, they presented normal food consumption and normal behaviors. The changes in body weight, fat content and body fat ratio (the ratio of the fat around kidney and testis to body weight) of each group before and after the experiment are listed in Table 4.

**Table 4: The influence of the extract of lingonberry on body weight and fat content of nutritional obese rats**

| group | | body weight (g) | | fat content around kidney and testes (g) | body fat ratio |
|---|---|---|---|---|---|
| | | before experiment | after 8 week feeding | | |
| control groups | normal feed | 185.30±8.51 | 408.40±25.98* | 10.09±3.99 | 2.30±0.88* |
| | high fat feed | 187.20±7.93 | 465.10±46.98 | 17.77±7.32 | 3.14±1.15 |
| administration groups | high dose | 186.33±8.10 | 428.36±41.10* | 8.92±2.72* | 2.12±0.56* |
| | middle dose | 188.42±6.67 | 448.33±46.54* | 11.92±3.19* | 2.62±0.72* |
| | low dose | 183.56±7.89 | 431.73±30.21* | 11.70±3.45* | 2.58±0.70* |

| | | | | | |
|---|---|---|---|---|---|
| *compared with high fat feed control group, P<0.05 | | | | | |

It can be seen that the extract of lingonberry can inhibit remarkably overnutrition-induced overweight, and decrease the fat around tissues and body fat ratio while presenting no influence to food consumption of test animals. Hence, the extract of lingonberry can be used as health food for anti-obesity.

### Example 15: The influence of the extract of lingonberry on SOD activity and HYP (hydroxyproline) content in skin of the photoaging mouse model

50 mice (*Mus musculus* KM) weighing 15~20g were randomly divided into 5 groups: normal control group, model control group, and 3 test groups including high, middle and low dose group. The groups except the normal control group were shaved off back hairs and irradiated with UVA lamp everyday to form a photoaging model. The high, middle and low dose groups were administrated orally with 200, 100 and 50 mg/kg bw of the first extract of lingonberry (prepared according to example 7, containing 5.2% of anthocyanin, 22.3% of proanthocyanidin and 0.5% of resveratrol) respectively, while the control group administrated with distilled water of the same volume. After 8 weeks, the mice were killed and immediately taken off the skin in the shaved area, which was tested for local SOD activity and HYP content by biochemical method. The results were listed in Table 5.

**Table 5: The influence of the extract of lingonberry on SOD activity and HYP (hydroxyproline) content of photoaging mice model**

| | SOD (NU/mg prot) | HYP (nmol/mg prot) |
|---|---|---|
| normal control group | 43.15±7.21* | 2.85±0.69* |
| model control group | 12.99±2.16 | 1.11±0.24 |
| high dose group | 22.63±3.74* | 2.68±0.65* |
| middle dose group | 17.85±4.33 | 1.82±0.39 |
| low dose group | 16.38±4.38 | 1.56±0.50 |

| | | |
|---|---|---|
| *compared with model control group, P<0.05 | | |

It can be seen that the extract of lingonberry can inhibit skin's aging, improve SOD activity and HYP content in the skin, and keep skin's energetic and elastic. Therefore, it can be used as health foods and cosmetics for beauty functions.

## Claims

1. An extract of lingonberry, which is comprised of 5-35wt% of anthocyanin, 20-95 wt% of proanthocyanidin, less than 5 wt% of resveratrol and the balance of other components.

2. The extract of lingonberry according to claim 1, comprised of 5-20 wt% of anthocyanin, 50-95 wt% of proanthocyanidin, less than 2 wt% of resveratrol and the balance of other components.

3. The extract of lingonberry according to claim 2, wherein said anthocyanin substantively comprising cyanidin - 3-galactose, cyanidin - 3-glucose and cyanidin-3-arabinose.

4. An extract composition of lingonberry comprised of:
a first extract of lingonberry comprised of 5-20 wt% of anthocyanin, 50-95 wt% of proanthocyanidin, less than 2 wt% of resveratrol and the balance of other components, and
a second extract of lingonberry containing 60-98 wt% of resveratrol.

5. The extract of lingonberry according to claim 1, wherein the extract of lingonberry is an extract product obtainable by a process comprising the steps of:
(a) extracting the fruit of lingonberry with a first solvent selected from alcohol, aqueous alcohol solution, water, aqueous acid solution, aqueous enzyme-containing solution, or aqueous acid-alcohol solution, and then isolating to obtain first extract solution;
(b) evaporating said first extract solution to obtain a crude first extract ;
(c) purifying said crude first extract to obtain a first extract product.

6. The extract of lingonberry according to claim 5, wherein in step (a), said first solvent is an aqueous acid solution or an aqueous acid-alcohol solution containing no more than 10 wt% of hydrochloric acid, sulphuric acid, acetic acid or citric acid, or a aqueous solution containing 0.05-0.2 wt% of cellulase or pectinase.

7. The extract of lingonberry according to claim 5, wherein, in step (a),
said first solvent is an aqueous acid solution or an aqueous acid-alcohol solution, and said extraction is performed under a temperature of 20-50 °C; or
said first solvent is an aqueous alcohol solution, and said extraction is performed under a temperature of 40-90 °C; or
said first solvent is an aqueous enzyme-containing solution, and said extraction is performed under a temperature of 30-60 °C.

8. The extract of lingonberry according to claim 5, wherein in step (c),
said crude first extract is purified with an absorptive resin or an ion exchange resin, including transferring said crude first extract into an absorptive resin column or an ion exchange resin column, eluting with 30-95% ethanol, concentrating the obtained ethanol solution and drying to obtain a first extract of lingonberry; or
said crude first extract is purified by an ultra-filtration membrane, including passing said crude first extract through an ultrafiltration membrane, concentrating the obtained retentate or passing it by a reverse osmosis membrane, and drying the obtained concentrated solution to obtain the first extract of lingonberry; or
the purification is performed combining the above two methods, including passing said crude first extract through an ultrafiltration membrane, purifying the obtained retentate with a resin column, and drying to obtain the first extract of lingonberry; or purifying the crude first extract by a resin column, then treating with an ultrafiltration membrane, and drying to obtain the first extract of lingonberry.

9. The extract composition of lingonberry according to claim 4, wherein the first extract of lingonberry comprised of 5-20 wt% of anthocyanin, 50-95 wt% of proanthocyanidin, less than 2 wt% of resveratrol and the balance of other components is obtainable by a process comprising the steps of:
(a) extracting the fruit of lingonberry with a first solvent selected from alcohol, aqueous alcohol solution, water, aqueous acid solution, aqueous enzyme-containing solution, or aqueous acid-alcohol solution, and then isolating to obtain a first extract solution;
(b) concentrating said first extract solution to obtain a crude first extract ;
(c) purifying said crude first extract to obtain a first extract; and
wherein the second extract of lingonberry containing 60-98 wt% of resveratrol is obtainable by a process comprising the steps of:
(d) re-extracting the fruit of lingonberry, which has extracted with the first solvent, with a second solvent to obtain a second extract solution, wherein said second extract solution is selected from ethyl acetate, benzene, toluene, chloroform, dichloromethane, n-hexane, acetone, methanol, ethanol, and the mixture thereof, or 60-95wt% methanol or ethanol aqueous solution, or aqueous enzyme-containing solution containing 0.01-0.3 wt% of cellulase or pectase;
(e) concentrating said second extract solution to obtain a crude second extract;
(f) purifying said crude second extract to obtain a second extract of lingonberry in which the main component is resveratrol.

10. The extract composition of lingonberry according to claim 9, wherein
in step (a) said first solvent is an aqueous acid solution or an aqueous acid-alcohol solution, and said extraction is performed under 20-50 °C; or said first solvent is aqueous alcohol solution, and said extraction is performed under 40-90 °C; or said first solvent is the enzyme aqueous solution, and said enzymolysis is performed under the temperature of 30-60 °C;
in step (b), said first extract solution is concentrated to obtain the crude first extract;
in step (c),
said crude first extract is transferred into an absorptive resin column or an ion exchange resin column, eluted with 30-95% ethanol, wherein the obtained ethanol solution is concentrated and dried to obtain a first extract of lingonberry; or
said crude first extract is purified by an ultra-filtration membrane, including passing said crude first extract through an ultrafiltration membrane, concentrating the obtained retentate or passing it by a reverse osmosis membrane, and drying the obtained concentrated solution to obtain the first extract of lingonberry; or
the purification is performed combining the above two methods, including passing said crude first extract through an ultrafiltration membrane, purifying the obtained retentate with a resin column, and drying to obtain the first extract of lingonberry; or purifying the crude first extract by a resin column, then treating with an ultrafiltration membrane, and drying to obtain the first extract of lingonberry;
in step (d), said re-extracting is performed under light-proof and inert atmosphere to obtain said second extract solution;
in step (e), said second extract solution is concentrated to obtain said crude second extract;
in step (f), said crude second extract is purified by silica gel column chromatography with n-hexane, ethyl acetate, acetone, chloroform, dichlormethane, methanol, ethanol or a mixture solvent thereof to obtain said second extract of lingonberry.

## Patentansprüche

1. Preiselbeerextrakt, bestehend aus 5 - 35 Gew.-% Anthocyanin, 20 - 95 Gew.-% Proanthocyanidin, weniger als 5 Gew.-% Resveratrol und als Rest andere Bestandteile.

2. Preiselbeerextrakt nach Anspruch 1,
bestehend aus 5 - 20 Gew.-% Anthocyanin, 50 - 95 Gew.-% Proanthocyanidin, weniger als 2 Gew.-% Resveratrol und als Rest andere Bestandteile.

3. Preiselbeerextrakt nach Anspruch 2,
wobei das Anthocyanin im Wesentlichen Cyanidin-3-Galactose, Cyanidin-3-Glucose und Cyanidin-3-Arabinose umfasst.

4. Extraktzusammensetzung der Preiselbeere bestehend aus:
einem ersten Preiselbeerextrakt bestehend aus 5 - 20 Gew.-% Anthocyanin, 50 - 95 Gew.-% Proanthocyanidin, weniger als 2 Gew.-% Resveratrol und als Rest andere Bestandteile, und
einem zweiten Preiselbeerextrakt enthaltend 60 - 98 Gew.-% Resveratrol.

5. Preiselbeerextrakt nach Anspruch 1,
wobei das Preiselbeerextrakt ein Extrakterzeugnis ist erhältlich durch ein Verfahren umfassend die Schritte:
(a) Extrahieren der Frucht der Preiselbeere mit einem ersten Lösungsmittel ausgewählt aus Alkohol, wässriger Alkohollösung, Wasser, wässriger Säurelösung, wässriger Enzym enthaltender Lösung oder wässriger saurer Alkohollösung und dann Abtrennen, um eine erste Extraktlösung zu erhalten;
(b) Verdampfen der ersten Extraktlösung, um ein erstes Rohextrakt zu erhalten;
(c) Reinigen des ersten Rohextrakts, um ein erstes Extraktprodukt zu erhalten.

6. Preiselbeerextrakt nach Anspruch 5,
wobei in Schritt (a) das erste Lösungsmittel eine wässrige Säurelösung oder eine wässrige saure Alkohollösung ist, welche nicht mehr als 10 Gew.-% Salzsäure, Schwefelsäure, Essigsäure oder Zitronensäure enthält oder eine wässrige Lösung enthaltend 0,05 - 0,2 Gew.-% an Cellulase oder Pektinase.

7. Preiselbeerextrakt nach Anspruch 5,
wobei in Schritt (a) das erste Lösungsmittel eine wässrige Säurelösung oder eine wässrige saure Alkohollösung ist und die Extraktion bei einer Temperatur von 20 - 50°C durchgeführt wird; oder das erste Lösungsmittel eine wässrige Alkohollösung ist und die Extraktion bei einer Temperatur von 40 - 90°C durchgeführt wird; oder das erste Lösungsmittel eine wässrige Enzym enthaltende Lösung ist und die Extraktion bei einer Temperatur von 30 - 60°C durchgeführt wird.

8. Preiselbeerextrakt nach Anspruch 5,
wobei in Schritt (c)
das erste Rohextrakt mit einem Absorptionsharz oder einem Ionenaustauschharz gereinigt wird, umfassend ein Überführen des ersten Rohextrakts auf eine Absorptionsharzsäule oder eine Ionenaustauschharzsäule, Eluieren mit 30 - 95 % Ethanol, Aufkonzentieren der erhaltenen Ethanollösung und Trocknen, um ein erstes Preiselbeerextrakt zu erhalten; oder
das erste Rohextrakt mittels einer Ultrafiltrationsmembran gereinigt wird, umfassend Durchführen des ersten Rohextrakts durch eine Ultrafiltrationsmembran, Aufkonzentrieren des erhaltenen Retentats oder
Vorbeiführen an einer Umkehrosmosemembran und Trocknen der erhaltenen aufkonzentrierten Lösung, um ein erstes Preiselbeerextrakt zu erhalten; oder
die Reinigung als Kombination der beiden oben angegebenen Verfahren durchgeführt wird, umfassend Durchführen des ersten Rohextrakts durch eine Filtrationsmembran, Reinigen des erhaltenen Retentats mit einer Harzsäule und Trocknen, um das erste Preiselbeerextrakt zu erhalten; oder Reinigen des ersten Rohextrakts durch eine Harzsäule und dann Behandeln mit einer Ultrafiltrationsmembran und Trocknen, um das erste Preiselbeerextrakt zu erhalten.

9. Extraktzusammensetzung der Preiselbeere nach Anspruch 4, wobei das erste Preiselbeerextrakt, das aus 5 - 20 Gew.-% Anthocyanin, 50 - 95 Gew.-% Proanthocyanidin, weniger als 2 Gew.-% Resveratrol und als Rest andere Bestandteile besteht, erhältlich ist durch ein Verfahren umfassend die Schritte:
(a) Extrahieren der Frucht der Preiselbeere mit einem ersten Lösungsmittel, ausgewählt aus Alkohol, wässriger Alkohollösung, Wasser, wässriger Säurelösung, wässriger Enzym enthaltender Lösung oder wässriger saurer Alkohollösung und dann Abtrennen, um eine erste Extraktlösung zu erhalten;
(b) Aufkonzentrieren der ersten Extraktlösung, um ein erstes Rohextrakt zu erhalten;
(c) Reinigen des ersten Rohextrakts, um ein erstes Extrakt zu erhalten; und worin das zweite Preiselbeerextrakt 60 - 98 Gew.-% Resvertraol enthält und erhältlich ist durch ein Verfahren umfassend die Schritte:
(d) erneutes Extrahieren der Frucht der Preiselbeere, welche mit dem ersten Lösungsmittel extrahiert wurde, mit einem zweiten Lösungsmittel, um eine zweite Extraktlösung zu erhalten, wobei die zweite Extraktlösung ausgewählt ist aus Ethylacetat, Benzol, Toluol, Chloroform, Dichlormethan, n-Hexan, Aceton, Methanol, Ethanol und deren Mischung oder 60 - 95 Gew.-% wässriger Methanol- oder Ethanollösung oder wässriger Enzym enthaltender Lösung, enthaltend 0,01 bis 0,3 Gew.-% Cellulase oder Pektinase;
(e) Aufkonzentrieren der zweiten Extraktlösung, um ein zweites Rohextrakt zu erhalten;
(f) Reinigen des zweiten Rohextrakts, um ein zweites Preiselbeerextrakt zu erhalten, in welchem Resveratrol die Hauptkomponente ist.

10. Extraktzusammensetzung der Preiselbeere nach Anspruch 9, wobei in Schritt (a) das erste Lösungsmittel eine wässrige Säurelösung oder eine wässrige Alkohollösung ist und die Extraktion bei 20 - 50°C durchgeführt wird; oder das erste Lösungsmittel eine wässrige Alkohollösung ist und die Extraktion bei 40 - 90°C durchgeführt wird; oder das erste Lösungsmittel eine Enzym enthaltende Lösung ist und die Enzymolyse bei einer Temperatur von 30 - 60°C durchgeführt wird;
in Schritt (b) die erste Extraktlösung aufkonzentriert wird, um das erste Rohextrakt zu erhalten;
in Schritt (c)
das erste Rohextrakt auf eine Absorptionsharzsäule oder eine Ionenaustauschharzsäule überführt wird, mit 30 - 95 % Ethanol eluiert wird, wobei die erhaltene Ethanollösung aufkonzentriert und getrocknet wird, um ein erstes Preiselbeerextrakt zu erhalten; oder
das erste Rohextrakt mit einer Ultrafiltrationsmembran gereinigt wird, umfassend Durchführen des ersten Rohextrakts durch eine Ultrafiltrationsmembran, Aufkonzentrieren des erhaltenen Retentats oder Vorbeiführen an einer Umkehrosmosemembran und Trocknen der erhaltenen konzentrierten Lösung, um das erste Preiselbeerextrakt zu erhalten; oder
die Reinigung als Kombination der beiden oben genannten Verfahren durchgeführt wird, umfassend Durchführen des ersten Rohextrakts durch eine Ultrafiltrationsmembran, Reinigen des erhaltenen Retentats mit einer Harzsäule und Trocknen, um das erste Preiselbeerextrakt zu erhalten; oder Reinigen des ersten Rohextrakts durch eine Harzsäule und dann Behandeln mit einer Ultrafiltrationsmembran und Trocknen, um das erste Preiselbeerextrakt zu erhalten;
in Schritt (d) die erneute Extraktion unter Lichtausschluss und inerter Atmosphäre durchgeführt wird, um die zweite Extraktlösung zu erhalten;
in Schritt (e) die zweite Extraktlösung aufkonzentriert wird, um das zweite Rohextrakt zu erhalten;
in Schritt (f) das zweite Rohextrakt mittels Kieselgelsäulenchromatographie mit n-Hexan, Ethylacetat, Aceton, Chloroform, Dichlormethan, Methanol, Ethanol oder einer Mischung dieser Lösungsmittel gereinigt wird, um das zweite Preiselbeerextrakt zu erhalten.

## Revendications

1. Extrait d'airelle lingonberry, qui est constitué de 5 à 35 % en poids d'anthocyanine, de 20 à 95 % en poids de proanthocyanidine, de moins de 5 % en poids de resvératrol et du pourcentage restant d'autres constituants.

2. Extrait d'airelle lingonberry suivant la revendication 1, constitué de 5 à 20 % en poids d'anthocyanine, de 50 à 95 % en poids de proanthocyanidine, de moins de 2 % en poids de resvératrol et du pourcentage restant d'autres constituants.

3. Extrait d'airelle lingonberry suivant la revendication 2, dans lequel ladite anthocyanine comprend substantiellement du cyanidine-3-galactose, du cyanidine-3-glucose et du cyanidine-3-arabinose.

4. Extrait d'airelle lingonberry, constitué :
d'un premier extrait d'airelle lingonberry constitué de 5 à 20 % en poids d'anthocyanine, de 50 à 95 % en poids de proanthocyanidine, de moins de 2 % en poids de resvératrol et du pourcentage restant d'autres constituants, et
d'un second extrait d'airelle lingonberry contenant 60 à 98 % en poids de resvératrol.

5. Extrait d'airelle lingonberry suivant la revendication 1, ledit extrait d'airelle lingonberry étant un produit consistant en extrait pouvant être obtenu par un procédé comprenant les étapes :
(a) d'extraction du fruit d'airelle lingonberry avec un premier solvant choisi entre un alcool, une solution aqueuse d'un alcool, l'eau, une solution aqueuse d'un acide, une solution aqueuse contenant une enzyme ou une solution aqueuse contenant un acide-alcool, et ensuite d'isolement pour obtenir une première solution d'extrait ;
(b) d'évaporation de ladite première solution d'extrait pour obtenir un premier extrait brut ;
(c) de purification dudit premier extrait brut pour obtenir un premier produit consistant en extrait.

6. Extrait d'airelle lingonberry suivant la revendication 5, dans lequel, dans l'étape (a), ledit premier solvant est une solution aqueuse d'un acide ou une solution aqueuse d'un acide-alcool ne contenant pas plus de 10 % en poids d'acide chlorhydrique, d'acide sulfurique, d'acide acétique ou d'acide citrique, ou bien une solution aqueuse contenant 0,05 à 0,2 % en poids de cellulase ou de pectinase.

7. Extrait d'airelle lingonberry suivant la revendication 5, dans lequel, dans l'étape (a),
ledit premier solvant est une solution aqueuse d'un acide ou une solution aqueuse d'acide-alcool, et ladite extraction est effectuée à une température de 20 à 50°C ; ou bien
ledit premier solvant est une solution aqueuse d'un alcool, et ladite extraction est effectuée à une température de 40 à 90°C ; ou bien
ledit premier solvant est une solution aqueuse contenant une enzyme, et ladite extraction est effectuée à une température de 30 à 60°C.

8. Extrait d'airelle lingonberry suivant la revendication 5, dans lequel, dans l'étape (c)
ledit premier extrait brut est purifié avec une résine absorbante ou une résine échangeuse d'ions, la purification comprenant le transfert dudit premier extrait brut dans une colonne de résine absorbante ou une colonne de résine échangeuse d'ions, l'élution avec de l'éthanol à 30-95 %, la concentration de la solution éthanolique obtenue et un séchage pour obtenir un premier extrait d'airelle lingonberry ; ou bien
ledit premier extrait brut est purifié avec une membrane d'ultrafiltration, la purification comprenant le passage dudit premier extrait brut à travers une membrane d'ultrafiltration, la concentration du rétentat obtenu ou le passage de ce rétentat à travers une membrane d'osmose inverse, et un séchage de la solution concentrée obtenue pour obtenir le premier extrait d'airelle lingonberry ; ou bien
la purification est effectuée en combinant les deux procédés précités, comprenant le passage dudit premier extrait brut à travers une membrane d'ultrafiltration, la purification du rétentat obtenu avec une colonne de résine, et un séchage pour obtenir le premier extrait d'airelle lingonberry ; ou bien
la purification du premier extrait brut avec une colonne de résine, puis le traitement avec une membrane d'ultrafiltration et un séchage pour obtenir le premier extrait d'airelle lingonberry.

9. Composition d'extrait d'airelles lingonberry suivant la revendication 4, dans lequel le premier extrait d'airelle lingonberry, constitué de 5 à 20 % en poids d'anthocyanine, de 50 à 95 % en poids de proanthocyanidine, de moins de 2 % en poids de resvératrol et du pourcentage restant d'autres constituants peut être obtenu par un procédé comprenant les étapes :
(a) d'extraction du fruit d'airelle lingonberry avec un premier solvant choisi entre un alcool, une solution aqueuse d'un alcool, l'eau, une solution aqueuse d'un acide, une solution aqueuse contenant une enzyme ou une solution aqueuse d'acide-alcool, et ensuite d'isolement pour obtenir une première solution d'extrait ;
(b) de concentration de ladite première solution d'extrait pour obtenir un premier extrait brut ;
(c) de purification dudit premier extrait brut pour obtenir un premier extrait ;
le second extrait d'airelle lingonberry contenant 60 à 98 % en poids de resvératrol pouvant être obtenu par un procédé comprenant les étapes :
(d) de réextraction du fruit d'airelle lingonberry, qui a été soumis à une extraction avec le premier solvant, avec un second solvant pour obtenir une seconde solution d'extrait, le solvant de ladite seconde solution d'extrait étant choisi entre l'acétate d'éthyle, le benzène, le toluène, le chloroforme, le dichlorométhane, le n-hexane, l'acétone, le méthanol, l'éthanol et leurs mélanges, ou une solution aqueuse à 60-95 % en poids de méthanol ou d'éthanol, ou une solution aqueuse contenant une enzyme, qui contient 0,01 à 0,3 % en poids de cellulase ou de pectase ;
(e) de concentration de ladite seconde solution d'extrait pour obtenir un second extrait brut ;
(f) de purification dudit second extrait brut pour obtenir un second extrait d'airelle lingonberry dans lequel le constituant principal est le resvératrol.

10. Composition d'extrait d'airelle lingonberry suivant la revendication 9, dans laquelle
dans l'étape (a), ledit premier solvant est une solution aqueuse d'un acide ou une solution aqueuse d'acide-alcool, et ladite extraction est effectuée à une température de 20 à 50°C ; ou ledit premier solvant est une solution aqueuse d'un alcool, et ladite extraction est effectuée à une température de 40 à 90°C ; ou bien ledit premier solvant est une solution aqueuse d'une enzyme et ladite enzymolyse est effectuée à une température de 30 à 60°C ;
dans l'étape (b), ladite première solution d'extrait est concentrée pour obtenir le premier extrait brut ;
dans l'étape (c)
ledit premier extrait brut est transféré dans une colonne de résine absorbante ou une colonne de résine échangeuse d'ions, en éluant avec de l'éthanol à 30-95 %, la solution éthanolique obtenue est concentrée et séchée pour obtenir un premier extrait d'airelle lingonberry ; ou
ledit premier extrait brut est purifié avec une membrane d'ultratfiltration, la purification comprenant le passage dudit premier extrait brut à travers une membrane d'ultrafiltration, la concentration du rétentat obtenu ou le passage de ce rétentat à travers une membrane d'osmose inverse, et le séchage de la solution concentrée obtenue pour obtenir le premier extrait d'airelle lingonberry ;
la purification est effectuée en combinant les deux procédés précités, comprenant le passage dudit premier extrait brut à travers une membrane d'ultrafiltration, la purification du rétentat obtenu avec une colonne de résine et un séchage pour obtenir le premier extrait d'airelle lingonberry ; ou la purification du premier extrait brut avec une colonne de résine, puis un traitement avec une membrane d'ultrafiltration et un séchage pour obtenir le premier extrait d'airelle lingonberry ;
dans l'étape (d) ladite réextraction est effectuée à l'abri de la lumière et sous atmosphère inerte pour obtenir ladite seconde solution d'extrait ;
dans l'étape (e), ladite seconde solution d'extrait est concentrée pour obtenir ledit second extrait brut ;
dans l'étape (f), ledit second extrait brut est purifié par chromatographie sur une colonne de gel de silice avec un solvant consistant en n-hexane, acétate d'éthyle, acétone, chloroforme, dichlorométhane, méthanol, éthanol ou un de leurs mélanges de solvants pour obtenir ledit second extrait d'airelle lingonberry.
